# EUROPÄISCHE PATENTSCHRIFT

(11) **EP 1 801 093 B1**
(45) Veröffentlichungstag und Bekanntmachung des Hinweises auf die Patenterteilung: **18.03.2009**
(21) Anmeldenummer: 06025546.0
(22) Anmeldetag: 11.12.2006
(51) Int. Cl.: C07C 51/36, C07C 59/52

(54) **Verfahren zur Herstellung von enantiomerenangereicherten 2-Alkoxy-3-Phenyl-Propion-Säuren**
Process for the Preparation of enantiomer-enriched 2-Alkoxy-3-Phenyl-Propionic Acid
Procédé pour la préparation de l'acide 2-alkoxy-3-phenylpropionique enrichi en enantiomère

(30) Priorität: 22.12.2005 DE 102005061472
(43) Veröffentlichungstag der Anmeldung: 27.06.2007
(73) Patentinhaber: Saltigo GmbH, 40764 Langenfeld (DE)
(72) Erfinder: Woltering, Michael, Dr., 40721 Hilden (DE); Bunlaksananusorn, Tanasri, Dr., 60100 Creil (FR); Gerlach, Arne Dr., 65843 Sulzbach (DE)
(74) Vertreter: Wichmann, Birgid

(56) Entgegenhaltungen:
- WO-A-00/26200
- WO-A-01/11072
- WO-A-20/05051882
- JP-A- 2001 261 612
- LINDERBERG M T ET AL: "Claisen condensation as a facile route to an alpha-alkoxy-cinnamate: Synthesis of ethyl (2S)-2-ethoxy-3-(4-hydroxyphenyl)propanoat e" ORGANIC PROCESS RESEARCH AND DEVELOPMENT, CAMBRIDGE, GB, Bd. 8, Nr. 6, 21. Oktober 2004 (2004-10-21), Seiten 838-845, XP002325851

## Beschreibung

Die vorliegende Erfindung betrifft ein Verfahren zur Herstellung von enantiomerenangereicherten gegebenenfalls substituierten 2-Alkoxy-3-phenylpropionsäuren durch asymmetrische Hydrierung von 2-Alkoxyzimtsäuren.

Substituierte 2-Alkoxy-3-phenylpropionsäuren stellen eine interessante Klasse von Wirkstoffen dar, die als Strukturmotiv insbesondere bei Agonisten der Peroxisom-Proliferator-Aktivierten Rezeptoren (PPAR) in den letzten Jahren zunehmend an Bedeutung gewonnen haben. Agonisten dieser Rezeptoren finden vorwiegend Verwendung in Substanzen zur Behandlung von Diabetes und Störungen des Lipidstoffwechsels (z.B. Ragaglitazar, Tesaglitazar, NNC 61-4655, u.a.). Demzufolge ist die Bereitstellung von möglichst enantiomerenreinen 2-Alkoxy-3-phenylpropionsäurebausteinen von besonderem Interesse für die Herstellung von solchen Wirksubstanzen.

Für die Herstellung substituierter 2-Alkoxy-3-phenylpropionsäuren sind konzeptionell verschiedene Verfahren beschrieben.

In WO-A- 2005/051 882 wird ein Verfahren zur Herstellung von optisch aktiven 3-(4-Hydrophenyl)-propionsäuren offenbart.

In J. Med. Chem. 2001, 44, 2675-2679, wird eine chromatographische Diastereomerentrennung der jeweiligen Säureamide beschrieben, die unter Zugabe chiraler enantioemerenreiner Amine aus der racemischen Säure gebildet wurden.

Des Weiteren besteht die Möglichkeit einer Racematspaltung mittels Diastereomerenkristallisation. So ist in WO-A 2004/00789 die Racematspaltung der racemischen substituierten 2-Alkoxy-3-phenylpropionsäuren mit chiralen Basen durch Diastereomerenkristallisation und in WO-A 2000/26200 die Spaltung der racemischen substituierten 2-Hydroxy-3-phenylpropionsäuren beschrieben. Solche Diastereomerenkristallisationen können auch einer chiral pool Synthese nachgeschaltet sein (vgl. WO-A 2000/26200, WO-A 2002/26425).

Auch kinetische Racematspaltungen racemischer substituierter 2-Alkoxy-3-phenylpropionsäuren durch enzymatische Verfahren wie Esterhydrolyse, Veresterung oder Umesterung sind in der Literatur beschrieben (vgl. WO-A 2001/11072 und WO-A 2001/11073). In Org. Proc Res. & Dev. 2003, 82 ist beispielsweise die Herstellung der (S)-3-(4-Hydroxyphenyl)-2-ethoxypropionsäure basierend auf enzymatischer Esterhydrolyse detailliert beschrieben.

Den vorangehenden Verfahren ist gemeinsam, dass anschließend an die eigentliche Synthese der 2-Alkoxy-3-phenylpropionsäuren die Racematspaltung in einem separaten Schritt erfolgen muss. Unter anderem auch zur wirtschaftlichen Optimierung solcher Synthesen wird daher gemeinhin nach Alternativen gesucht, die enantioselektive Syntheseschritte bereits beim Aufbau des Moleküls enthalten.

Eine solche Synthese ist beispielsweise in WO-A 2002/100813 mit einer enantioselektiven Reduktion der 2-Oxo-3-Phenylpropionsäure durch (+)-Chlor-diisopinocampheylboran zur 2-Hydroxy-Säure, die wiederum in Folge zum 2-Alkoxysäureester und dann zur 2-Alkoxysäure umgesetzt werden kann, beschrieben. Für die enantioselektive Hydrierung an substituierten Zimtsäuren sind weiterhin Beispiele für 2-Acylaminozimtsäureester beschrieben (Chem. Rev. 2003, 3029), darüber hinaus sind auch Beispiele für die Hydrierung von 2-Acyloxyzimtsäureestern sowie Benzylidenbernsteinsäurediestern bekannt (J. Am. Chem. Soc. 1998, 4345; Synthesis 1994, 1138; Synlett 2002, 837).

Diese Verfahren haben jedoch den Nachteil, dass zum Einen aufgrund der verwendeten Schutzgruppentechnik eine Reihe von Verfahrensschritten zur selektiven Einführung und Abspaltung der entsprechenden Schutzgruppen durchlaufen werden müssen und zum Anderen alle diese Verfahren über die entsprechenden Propionsäureester laufen, die in einem weiteren Schritt in die entsprechenden Propionsäuren überführt werden müssen.

Es bestand somit weiterhin Bedarf an einem Verfahren zur Herstellung von enantiomerenangereicherten 2-Alkoxy-3-phenylpropionsäuren, welches mit weniger Verfahrensschritten zu den gewünschten Produkten mit hohem Enantiomerenüberschuss führt.

Die Aufgabe, die der vorliegenden Erfindung zugrunde lag, bestand somit darin, ein solches Verfahren zur Herstellung von enantiomerenangereicherten 2-Alkoxy-3-phenylpropionsäuren aufzufinden.

Überraschend wurde nun gefunden, dass enantiomerenangereicherte 2-Alkoxy-3-phenylpropionsäuren durch asymmetrische Hydrierung von Alkoxyzimtsäuren in einem einfachen Verfahren ohne Schutzgruppentechnik mit hohem Enantiomerenüberschuss unter Verwendung eines Übergangsmetall-Hydrierkatalysators hergestellt werden können.

Gegenstand der vorliegenden Erfindung ist ein Verfahren zur Herstellung von enantiomerenangereicherten Verbindungen der allgemeinen Formel (I), 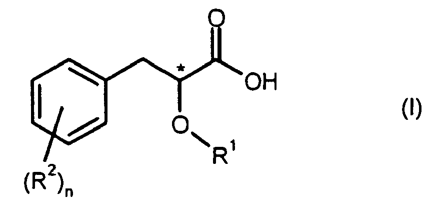 worin
- R¹: für einen gegebenenfalls substituierten C₁-C₁₈-Alkylrest, bevorzugt einen C₁-C₆-Alkylrest, einen gegebenenfalls substituierten C₄-C₂₄-Arylrest, bevorzugt einen C₆-C₂₄-Arylrest, oder einen gegebenenfalls substituierten C₅-C₁₈-Arylalkylrest steht,
- R²: unabhängig voneinander für OH, Halogen, Pseudohalogen, Amino, einen gegebenenfalls substituierten C₁-C₁₈-Alkylrest, bevorzugt einen C₁-C₆-Alkylrest, einen gegebenenfalls substituierten C₁-C₁₈-Alkoxyrest, bevorzugt einen C₁-C₆-Alkoxyrest, einen gegebenenfalls substituierten C₄-C₂₄-Arylrest, bevorzugt einen C₆-C₂₄-Arylrest, einen gegebenenfalls substituierten C₅-C₁₈-Arylalkylrest, einen gegebenenfalls substituierten C₁-C₁₈-Alkyl-Sulfonylrest, einen gegebenenfalls substituierten C₁-C₁₈-Alkyl-Carboxylrest, einen gegebenenfalls substituierten C₁-C₁₈-Alkyl-Carbonylrest, einen gegebenenfalls substituierten C₁-C₁₈-Mono- oder Dialkylaminorest, bevorzugt einen C₁-C₆-Mono- oder Dialkylaminorest, einen gegebenenfalls substituierten C₁-C₁₈-Alkylsulfonylaminorest oder einen gegebenenfalls substituierten C₁-C₁₈-Acylaminorest steht. Bevorzugt sind hierbei OH, Halogen, ein C₁-C₆-Alkylrest, ein C₁-C₆-Alkoxyrest oder ein C₅-C₁₈-Arylalkylrest, besonders bevorzugt ist OH.
- n: für 0 oder eine ganze Zahl von 1 bis 5, bevorzugt für 1, 2 oder 3, besonders bevorzugt für 1 steht,
dadurch gekennzeichnet, dass Verbindungen der allgemeinen Formel (II), 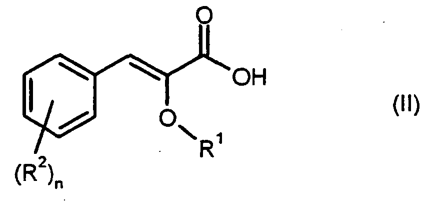 worin
R¹, R² und n die für die allgemeine Formel (I) angegebene Bedeutung haben,
in Gegenwart eines Übergangsmetall-Hydrierkatalysators in Form eines Katalysatorsystems mit Ruthernium-, Rhodium- oder Iridiumverbindungen der Oxidationsstufen Ru(II), Rh(I), Ir(I), jeweils in Kombination mit einem chiralen Diphosphin-Liganden ausgewählt aus (-)-2,2'-Dichlor-3,3'-dimethoxy-6,6'-bis-diphenylphosphanyl-biphenyl, (-)-3,3'-Bis-diphenylphosphanyl-[4,4']bi(dibenzofuranyl), und dem jeweiligen Enantiomeren enantioselektiv hydriert werden.

In einer bevorzugten Ausführungsform des erfindungsgemäßen Verfahrens werden die Verbindungen der allgemeinen Formel (II) aus Verbindungen der allgemeinen Formel (III), 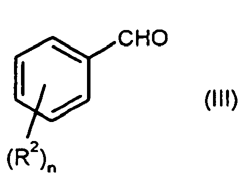 worin
R² und n die für die allgemeine Formel (I) genannte Bedeutung haben,
und Verbindungen der allgemeinen Formel (IV), 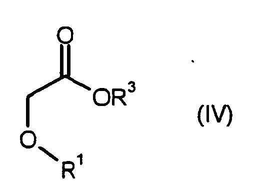 worin
- R³: für H oder einen gegebenenfalls substituierten C₁-C₁₈-Alkylrest, bevorzugt einen C₁-C₆-Alkylrest steht und
- R¹: die für die allgemeine Formel (I) genannte Bedeutung hat,
in einer Aldolreaktion in Gegenwart einer Base und gegebenenfalls anschließender Hydrolyse hergestellt hergestellt werden.

Bei dem mit * gekennzeichneten Kohlenstoff-Atom in der allgemeinen Formel (I) handelt es sich um ein asymmetrisches C-Atom. Enantiomerenangereicherte Verbindung der allgemeinen Formel (I) im Sinne der Erfindung bedeutet die enantiomerenreinen Verbindungen der allgemeinen Formel (I) in (*R*)- oder (*S*)-Konfiguration oder Mischungen der jeweiligen beiden Enantiomeren, in denen ein Enantiomer in einem Enantiomerenüberschuss, im Folgenden auch ee (_{"}enantiomeric excess") genannt, im Vergleich zum anderen Enantiomer vorliegt. Bevorzugt beträgt dieser Enantiomerenüberschuss 2 bis 100 % ee, besonders bevorzugt 60 % bis 100 % und ganz besonders bevorzugt 85 bis 100 %. Eine Definition des ee-Werts wird im Rahmen der Beispiele dieser Anmeldung angegeben.

Im Rahmen der Erfindung können alle oben stehenden und im Folgenden aufgeführten, allgemeinen oder in Vorzugsbereichen genannten Restedefinitionen, Parameter und Erläuterungen untereinander, also auch zwischen den jeweiligen Bereichen und Vorzugsbereichen in beliebiger Weise kombiniert werden.

Alkyl oder Alkoxy bedeutet jeweils unabhängig einen linearen, cyclischen, verzweigten oder unverzweigten Alkyl- oder Alkoxy-Rest. Gleiches gilt für den nichtaromatischen Teil eines Arylalkyl-Restes oder für den Alkylteil eines Alkyl-Sulfonyl-, Alkyl-Carboxyl-, Alkyl-Carbonyl, Mono- oder Dialkylamino- sowie Alkylsulfonylaminorestes.

C₁-C₆-Alkyl steht beispielsweise für Methyl, Ethyl, n-Propyl, iso-Propyl, n-Butyl, sec.-Butyl, tert.-Butyl, n-Pentyl, 1-Methylbutyl, 2-Methylbutyl, 3-Methylbutyl, neo-Pentyl, 1-Ethylpropyl, Cyclohexyl, Cyclopentyl, n-Hexyl, 1,1-Dimethylpropyl, 1,2-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl oder 1-Ethyl-2-methylpropyl, C₁-C₁₈-Alkyl darüber hinaus beispielsweise für n-Heptyl und n-Octyl, Pinakyl, Adamantyl, die isomeren Menthyle, n-Nonyl, n-Decyl, n-Dodecyl, n-Tridecyl, n-Tetradecyl, n-Hexadecyl oder n-Octadecyl.

**C₁-C₆-Alkoxy** steht beispielsweise für die den vorangehenden Alkylgruppen entsprechenden Alkoxygruppen, wie z.B. Methoxy, Ethoxy, n-Propoxy, iso-Propoxy, n-Butoxy, sec.-Butoxy, tert.-Butoxy, n-Pentoxy, n-Hexoxy etc.. Auch **C₁-C₁₈-Alkoxy** steht beispielsweise für die den vorangehenden Alkylgruppen entsprechenden Alkoxygruppen.

**C₁-C₁₈-Alkyl-Sulfonyl, C₁-C₁₈-Alkyl-Carboxyl, C₁-C₁₈-Alkyl-Carbonyl, C₁-C₆-Mono- oder Dialkylamino** bzw. **C₁-C₁₈-Mono- oder Dialkylamino** bzw. **C₁-C₁₈-Alkylsulfonylamino** steht beispielsweise für die den vorangehenden Alkylgruppen entsprechenden Alkyl-Sulfonyl-, Alkyl-Carboxyl-, Alkyl-Carbonyl-, Mono- oder Dialkylamino- bzw. Alkylsulfonylaminogruppen.

Aryl steht jeweils unabhängig für einen aromatischen Rest mit 4 bis 24 Gerüstkohlenstoffatomen, in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können, vorzugsweise jedoch für einen carbocyclischen aromatischen Rest mit 6 bis 24 Gerüstkohlenstoffatomen. Gleiches gilt auch für den Arylteil eines Aryl-carbonylrestes

Beispiele für **C₆-C₂₄-Aryl** sind Phenyl, o-, p-, m-Tolyl, Naphthyl, Phenanthrenyl, Anthracenyl oder Fluorenyl, Beispiele für heteroaromatisches **C₄-C₂₄-Aryl** in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können, sind beispielsweise Pyridinyl, Oxazolyl, Benzofuranyl, Dibenzofuranyl oder Chinolinyl.

Arylalkyl bedeutet jeweils unabhängig einen geradkettigen, cyclischem, verzweigten oder unverzweigten Alkyl-Rest nach obiger Definition, der einfach, mehrfach oder vollständig durch Aryl-Reste gemäß obiger Definition substituiert sein kann.

C₅-C₁₈-Arylalkyl steht beispielsweise für Benzyl oder (*R*)- oder (*S*)-1-Phenylethyl.

**Halogen** kann für Fluor, Chlor, Brom oder Iod stehen. **Pseudohalogen** kann beispielsweise für Cyanid, Cyanat oder Thiocyanat stehen.

Als mögliche Substituenten für die Reste R¹, R² oder R³ kommen zahlreiche organische Gruppen in Frage, beispielsweise Alkyl-, Cycloalkyl-, Aryl-, Halogen-, Hydroxyl-, Ether-, Thioether-, Disulfid-, Sulfoxid-, Sulfonsäure-, Sulfonat-, Amino-, Aldehyd-, Keto-, Carbonsäureester-, Carbonat-, Carboxylat, Cyano-, Alkylsilan- und Alkoxysilangruppen sowie Carboxylamidgruppen.

Bevorzugt steht R¹ für einen gegebenenfalls substituierten C₁-C₆-Alkylrest, in einer bevorzugten Ausführungsform des erfindungsgemäßen Verfahrens für Methyl.

Bevorzugt steht R² unabhängig voneinander für OH, F, Cl, Br, CN, einen gegebenenfalls substituierten C₁-C₆-Alkylrest, einen gegebenenfalls substituierten C₆-C₂₄-Arylrest oder einen gegebenenfalls substituierten C₅-C₁₈-Arylalkylrest, in einer bevorzugten Ausführungsform des erfindungsgemäßen Verfahrens für OH.

Bevorzugt steht R³ für H oder einen gegebenenfalls substituierten C₁-C₆-Alkylrest, in einer bevorzugten Ausführungsform des erfindungsgemäßen Verfahrens für Methyl.

R² kann sich in der allgemeinen Formeln (III) in ortho-, meta- oder para-Stellung zur Aldehydgruppe und somit in den Verbindungen der allgemeinen Formeln (I) und (II) ebenfalls in der entsprechenden Stellung zum Propionylsubstituenten befmden. In bevorzugten Ausführungsformen befindet sich R² in para-Stellung.

Als Übergangsmetall-Hydrierkatalysator wird erfindungsgemäß ein Katalysatorsystem in Form eines Katalysatorsystems mit Ruthernium-, Rhodium-oder Iridiumverbindungen der Oxidationsstufen Ru(II), Rh(I), Ir(I), jeweils in Kombination mit einem chiralen Diphosphin-Liganden ausgewählt aus (-)-2,2'-Dichlor-3,3'-dimethoxy-6,6'-bis-diphenylphosphanyl-biphenyl, (-)-3,3'-Bis-diphenylphosphanyl-[4,4']bi(dibenzofuranyl), und dem jeweiligen Enantiomeren

Im Rahmen der Erfindung kann der Übergangsmetall-Hydrierkatalysator entweder als isolierter Komplex eingesetzt werden oder in situ erzeugt werden. Bevorzugt wird der Übergangsmetall-Hydrierkatalysator in situ erzeugt. In letzterem Falle werden eine oder mehrere geeignete Übergangsmetallverbindung(en) und der oder die Liganden in einem geeigneten Lösungsmittel gegebenenfalls in Inertgasatmosphäre zusammengegeben und die Mischung gegebenenfalls anschließend erhitzt.

Übergangsmetallverbindung(en) und Ligand(en) sind im Katalysatorsystem je nach Übergangsmetallverbindung beispielsweise in einem Stoffrnengenverhältnis von 1 zu 0,8 bis 1 zu 4 (Übergangsmetallverbindung(en) zu Ligand(en)) enthalten. Für chirale Diphosphin-Liganden sind Verhältnisse von 1 zu 0,9 bis 1 zu 2,5 bevorzugt.

Die Menge des eingesetzten Übergangsmetall-Hydrierkatalysator liegt im Rahmen der Erfindung beispielsweise zwischen 0.001 und 20 mol%, bevorzugt zwischen 0.01 und 10 mol% und besonders bevorzugt zwischen 0,1 und 5 mol% bezogen auf die Stoffmenge der Verbindung der allgemeinen Formel (II).

Bevorzugt wird die enantioselektive Hydrierung in Gegenwart wenigstens eines protischen Lösungsmittels durchgeführt. Als geeignete protische Lösungsmittel kommen beispielsweise Alkohole, wie z.B. wie Methanol, Ethanol, n-Propanol, Isoproylalkohol, n-Butanol, sek-Butanol, tert.-Butanol, tert-Amylalkohol, sec-Amylalkohol in Frage. Diese können allein oder in Kombination mit anderen geeigneten Lösungsmitteln verwendet werden. Als andere geeignete Lösungsmittel kommen Carbonsäureester, wie z.B. Ethylacetat, Isopropylacetat, n-Propylacetat, n-Butylacetat, halogenierte aliphatische Kohlenwasserstoffe, wie z.B. Dichlormethan, 1,2-Dichlorethan, Chloroform, Tetrachlormethan, cyclische oder aliphatische Ether, wie z.B. Tetrahydrofuran, Dioxan, tert-Butylmethylether, cyclische oder aliphatische Kohlenwasserstoffe, wie z.B. Cyclohexan, Pentan, Hexan, Heptan, aromatischen Kohlenwasserstoffe, wie z.B. Toluol, Xylol, Cumol, Cymol, oder Mischungen aus diesen bzw. enthaltend einen oder mehrere von diesen in Frage.

Bevorzugt wird die enantioselektive Hydrierung bei Temperaturen von 10 bis 120°C, besonders bevorzugt von 20 bis 100°C, in bevorzugten Ausführungsformen von 40 bis 80°C durchgeführt. Die Reaktionszeit beträgt bevorzugt mehrere Stunden, besonders bevorzugt 0,2 bis 48 h, ganz besonders bevorzugt 10 bis 30 h.

Die Durchführung der enantioselektive Hydrierung unter Schutzgasatmosphäre, wie z.B. Stickstoff- oder Argonatmosphäre, kann von Vorteil sein, ist jedoch nicht zwingend erforderlich.

Weiterhin bevorzugt wird die enantioselektive Hydrierung in Abhängigkeit des eingesetzten Übergangsmetall-Hydrierkatalysators bei 1 bis 200 bar Wasserstoffdruck durchgeführt. Beispielsweise kann bei Verwendung eines eine Rhodiumverbindung enthaltenden Katalysatorsystems ein niedrigerer Wasserstoffdruck von beispielsweise 1 bis 50 bar zum optimalen Umsatz fuhren, wohingegen für ein eine Iridiumverbindung enthaltendes Katalysatorsystem unter Umständen ein höherer Wasserstoffdruck von beispielsweise 1 bis 100 bar und für ein eine Rutheniumverbindung enthaltendes Katalysatorsystem unter Umständen ein Wasserstoffdruck von 5 bis 180 bar von Vorteil hinsichtlich des Reaktionsverlaufs sein kann.

Gegebenenfalls können basische Zusätze wie tertiäre Amine oder Alkalimetall-C₁-C₁₈Alkoxide, wie beispielsweise Triethylamin oder Natriummethanolat, der enantioselektiven Hydrierung zugesetzt werden. Bevorzugt erfolgt dieser Zusatz in einer Menge von 0,5 bis 2 Äquivalenten, bevorzugt in nahezu äquimolaren Mengen, bezogen auf die Stoffmenge eingesetzten Zimtsäurederivates.

Als Base in der Aldolreaktion können beispielsweise Alkali- oder Erdalkalimetall-C₁-C₁₈-Alkoxide, wie z.B. Natrium- oder Kaliummethylat, -ethylat, -n-, -sec- oder -tert-butylat, oder gegebenenfalls substituierte Alkali- oder Erdalkalimetall-C₁-C₁₈Amide, wie z.B. Lithiumdiisopropylamid oder Hexamethyldisilazan, eingesetzt werden. Besonders bevorzugt wird Natriummethylat eingesetzt. Die Base wird bevorzugt im Überschuss eingesetzt. Besonders bevorzugt sind Mengen von 2 bis 6 Äquivalenten, ganz besonders bevorzugt von 4 bis 5 Äquivalenten, bezogen auf die Stoffmenge der Verbindung der allgemeinen Formel (III).

Die Aldolreaktion wird bevorzugt in Gegenwart wenigstens eines Lösungsmittels durchgeführt. Als Lösungsmittel kommen beispielsweise Alkohole, wie z.B. wie Methanol, Ethanol, n-Propanol, Isoproylalkohol, n-Butanol, sek-Butanol, tert.-Butanol, tert-Amylalkohol, sec-Amylalkohol, cyclische oder offenkettige Ether, wie z.B. Diethylether, Tert-butyl-methylether, Tetrahydrofuran, Methyl-Tetrahydrofuran, Dioxan oder Tetrahydropyran, aromatische Kohlenwasserstoffe, wie z.B. Toluol, Xylol, Cumol, Cymol, aliphatische Kohlenwasserstoffe, wie z.B. n-Pentan, n-Hexan, n-Heptan, Isohexan, cyclische Kohlenwasserstoffe, wie z.B. Cyclopentan, Cyclohexan, Methylcyclohexan, oder Mischungen aus diesen oder enthaltend ein oder mehrere von diesen in Frage. Besonders bevorzugt sind Alkohole oder Mischungen enthaltend Alkohole.

Bevorzugt wird die Aldolreaktion bei Temperaturen von 10 bis 120°C, besonders bevorzugt von 20 bis 100°C, in bevorzugten Ausführungsformen von 40 bis 80°C durchgeführt. Die Reaktionszeit beträgt bevorzugt mehrere Stunden, besonders bevorzugt 0,2 bis 48 h, ganz besonders bevorzugt 1 bis 24 h. Bevorzugt wird die Reaktionslösung solange gerührt, bis die Umsetzung der Verbindung der allgemeinen Formel (III) nahezu vollständig ist.

Die Aldolreaktion kann bei normalem, erhöhtem oder vermindertem Druck durchgeführt werden, beispielsweise im Bereich von 0,5 bis 5 bar. Im Allgemeinen wird sie bei Normaldruck durchgeführt.

Die Durchführung der Aldolreaktion unter Schutzgasatmosphäre, wie z.B. Stickstoff- oder Argonatmosphäre, kann von Vorteil sein, ist jedoch nicht zwingend erforderlich.

Nach erfolgter nahezu vollständiger Umsetzung der Verbindung der allgemeinen Formel (III) kann - sofern R³ von H verschieden ist - zur Hydrolyse und Herstellung der Verbindung der allgemeinen Formel (II) zur Reaktionslösung beispielsweise Wasser zugegeben werden. Die Hydrolyse kann jedoch auch nach vorheriger Isolierung des entsprechenden Esters der Verbindnung der allgemeinen Formel (II) separat erfolgen. Bevorzugt wird die Hydrolyse jedoch ohne Isolierung des Esters direkt in der Reaktionslösung durchgeführt. Bevorzugt wird die Hydrolyse bei Temperaturen von 10 bis 120°C, besonders bevorzugt von 20 bis 100°C, in bevorzugten Ausführungsformen von 30 bis 80°C durchgeführt. Während der Hydrolyse kann es von Vorteil sein, einen Teil des Lösungsmittels abzudestillieren. Anschließend kann die Reaktionslösung gegebenenfalls nach vorherigen Abkühlens vorzugsweise auf Raumtemperatur zur Ausfällung der Verbindung der allgemeinen Formel (II) angesäuert werden. Hierzu werden vorzugsweise Mineralsäuren verwendet. Geeignete Mineralsäuren sind dem Fachmann bekannt, bevorzugt wird konzentrierte wässrige Salzsäure verwendet. Gegebenenfalls wird die angesäuerte Reaktionslösung zur Vervollständigung der Ausfällung noch einige Zeit, vorzugsweise zwischen 15 Minuten und 10 Stunden bei Raumtemperatur gerührt und die Verbindung der allgemeinen Formel (II) anschließend isoliert. Die Isolation kann nach üblichen, dem Fachmann bekannten Methoden, wie z.B. Filtration erfolgen.

Die Verbindungen der allgemeinen Formel (IV) werden in der Aldolreaktion bevorzugt in einer Menge von 1 bis 4 Äquivalenten - bezogen auf die Stoffmenge der Verbindung der allgemeinen Formel (III) - eingesetzt. Besonders bevorzugt werden die Verbindungen der allgemeinen Formel (IV) im Überschuss eingesetzt; in einer bevorzugten Ausführungsform werden 2 bis 3 Äquivalente der Verbindungen der allgemeinen Formel (IV) bezogen auf die Stoffinenge der Verbindung der allgemeinen Formel (III) eingesetzt.

Die Verbindungen der allgemeinen Formeln (III) und (IV) sind in der Regel kommerziell erhältlich oder mittels allgemein bekannter Herstellverfahren aus kommerziell erhältlichen Vorläufern darstellbar. In einer bevorzugten Ausführungsform werden als Verbindung der allgemeinen Formel (III) 4-Hydroxybenzaldehyd und als Verbindung der allgemeinen Formel (IV) Methylmethoxyacetat eingesetzt.

Die Verbindungen der allgemeinen Formel (I) können entweder in Form der freien Säuren oder aber in Form von Salzen der allgemeinen Formel (V), 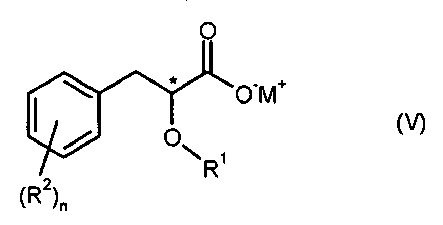 worin
- M⁺: für ein Alkalimetallkation oder Ammonium, bevorzugt für Na⁺ steht und
- R¹, R² und n: die für die allgemeine Formel (I) angegebene Bedeutung haben,
isoliert werden.

Die Herstellung der Salze der allgemeinen Formel (V) kann nach vorheriger Zwischenisolierung der Verbindungen der allgemeinen Formel (I) oder aber ohne eine solche vorherige Zwischenisolierung erfolgen. Gegebenenfalls kann ein Lösungsmittelwechsel vor der Herstellung der Salze der allgemeinen Formel (V) von Vorteil sein, was jedoch im Rahmen der Erfindung nicht als Zwischenisolierung der Verbindungen der allgemeinen Formel (I) zu verstehen ist. Hierzu wird zunächst das für die enantioselektive Hydrierung verwendete Lösungsmittel gegebenenfalls unter vermindertem Druck entfernt und der verbleibende Rückstand ohne weitere Reinigungsschritte in einem weiteren Lösungsmittel aufgenommen. Dabei kann das weitere Lösungsmittel ebenfalls Lösungsmittel enthalten, die auch im Lösungsmittel für die enantioselektive Hydrierung enthalten waren.

Geeignete weitere Lösungsmittel für die Herstellung der Salze der allgemeinen Formel (V) sind beispielsweise Alkohole, wie z.B. Methanol, Ethanol, n-Propanol, Isopropanol, n-Butanol, Carbonsäureester, wie z.B. Ethylacetat, Isopropylacetat, n-Propylacetat, n-Butylacetat, Ketone, wie z.B. Aceton, Butylmethylketon, Nitrile, wie z.B. Acetonitril, Propionitril, Butyronitril, cyclische oder offenkettige Ether, wie z.B. Diethylether, Dibutylether, tert.-Butylmethylether, Tetrahydrofuran, Dioxan, oder Lösungsmittel enthaltend einen oder mehrere von diesen. Bevorzugt sind hierbei Carbonsäureester oder Lösungsmittelgemische diese enthaltend.

Die Salze der allgemeinen Formel (V) werden in Gegenwart eines Alkalimetall- oder Ammoniumsalzes aus der entsprechenden Lösung ausgefällt bzw. auskristallisiert. Als geeignete Alkalimetall- oder Ammoniumsalze kommen beispielsweise Alkalimetall- oder Ammoniumsalze von Carbonsäuren, wie z.B. Acetate, bevorzugt deren Natriumsalze in Frage. Besonders bevorzugt wird Natriumacetat eingesetzt. Die Alkalimetall- oder Ammoniumsalze können vor der Zugabe zu den Verbindungen der allgemeinen Formel (I) vorher in einem geeigneten Lösungsmittel vollständig oder teilweise gelöst oder suspendiert werden. Solche geeigneten Lösungsmittel sind beispielsweise Alkohole, wie z.B. wie Methanol, Ethanol, n-Propanol, Isoproylalkohol, n-Butanol, sek-Butanol, tert.-Butanol, tert-Amylalkohol, sec-Amylalkohol, oder Carbonsäureester, wie z.B. Ethylacetat, Isopropylacetat, n-Propylacetat, n-Butylacetat, Mischungen aus diesen oder Lösungsmittel enthaltend diese.

Die Isolierung der Salze der allgemeinen Formel (V) kann nach bekannten Verfahren, wie z.B. durch Filtration erfolgen. Gegebenenfalls kann durch eine nachfolgende Umkristallisation die Enantiomerenreinheit der Salze der allgemeinen Formel (V) weiter gesteigert werden. Geeignete Lösungsmittel für eine solche Umkristallisation sind beispielsweise Alkohole, wie z.B. wie Methanol, Ethanol, n-Propanol, Isoproylalkohol, n-Butanol, sek-Butanol, tert.-Butanol, tert-Amylalkohol, sec-Amylalkohol, oder Mischungen aus diesen oder enthaltend einen oder mehrere von diesen.

Die nach dem erfindungsgemäßen Verfahren hergestellten Verbindungen der allgemeinen Formel (I) gegebenenfalls in Form der entsprechenden Salze der allgemeinen Formel (V) stellen - wie eingangs bereits erwähnt - beispielsweise pharmazeutische Wirkstoffe dar, die als Strukturmotiv insbesondere bei Agonisten der Peroxisom-Proliferator-Aktivierten Rezeptoren (PPAR) in den letzten Jahren zunehmend an Bedeutung gewonnen haben. Das erfindungsgemäße Verfahren liefert im Vergleich zu bekannten Verfahren einen deutlich einfacheren Zugang zu den enantiomerenangereicherten Verbindungen der allgemeinen Formel (I), da zum Einen die aufwändige Schutzgruppentechnik entfällt und zum Anderen auf direkten Weg die entsprechenden freien Propionsäuren erhalten werden. Dadurch werden beim erfindungsgemäßen Verfahren deutlich weniger Reaktionsschritte durchlaufen als bei bekannten Verfahren, wobei jedoch ebenso hohe Enantiomerenüberschüsse erzielt werden.

Die folgenden Beispiele dienen der beispielhaften Veranschaulichung der Erfindung und sind nicht als Beschränkung aufzufassen.

### Beispiele:

### Beispiel 1:

### Herstellung von Z-4'-Hydroxy-2-methoxyzimtsäure:

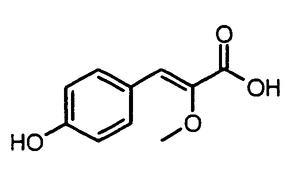

Eine Lösung von 150 g (1.20 mol) 4-Hydroxybenzaldehyd und 316 g (3.00 mol) Methylmethoxyacetat in 1,018 1 (5.39 mol) 30%iger Natriummethylat-Lösung in Methanol wurde 6 h unter Rückfluss erhitzt. Anschließend wurde das Gemisch auf 40°C abgekühlt und mit 1 Wasser versetzt. Aus dem Reaktionsgemisch wurden 760 ml Methanol abdestilliert und anschließend das Gemisch wieder auf Raumtemperatur abgekühlt. Die Reaktionslösung wurde mit 410 ml 37%iger wässriger Salzsäure auf pH 2 eingestellt, wobei das Produkt aus der Lösung ausgefällt wurde. Die Produktsuspension wurde noch 2 h bei Raumtemperatur gerührt, abfiltriert und der Filterrückstand im Vakuum bei 40°C über Nacht getrocknet. Es wurden 212.2 g (Gehalt: 92.4%, 83.8% d. Th.) des reinen Z-Produktes als gelblichen Feststoff erhalten.
¹H-NMR (400 MHz, d₆-DMSO): δ = 3.67 (s, 3H, O-CH₃); 6.79 (d, 2H, Ar-H); 6.85 (s, 1H, Ar-CH=); 7,61 (d, 2H, Ar-H); 9.82 (bs, 1H, Ar-OH); 12.67 (bs, 1H, COOH).
¹³C-NMR (75 MHz, d₄-MeOH): δ = 59.3 (O-CH₃); 116,5 (C_{Ar}-H); 125.8 (Ar-CH=); 126,2 (C_{Ar}-R); 133.1 (C_{Ar}-H); 144.8 (=C-OMe); 159.8 (C_{Ar}-OH); 168.1 (COOH).

### Beispiel 2:

### Herstellung von (S)-3-(4-Hydroxyphenyl)-2-methoxypropionsäure-Natrium-Salz durch Hydrierung mit Ruthenium-Katalysator:

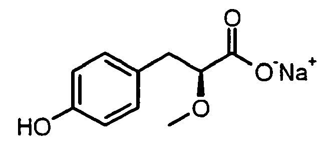

62.5 mg (1,0 mmol) [RuCl₂(p-Cymol)]₂ und 139,6 mg (2,1 mmol)) 2,2'-Dichlor-3,3'-dimethoxy-6,6'-bis-diphenylphosphanyl-biphenyl wurden in 150 ml entgastem Methanol suspendiert und 1 h unter Rückfluss erhitzt. Die entstandene Lösung wurde auf Raumtemperatur abgekühlt und zu einer Lösung von 40.46 g (200 mmol) Z-4'-Hydroxy-2-methoxyzimtsäure in 150 ml entgastem Methanol gegeben. Anschließend wurde das Gemisch in einen Autoklaven überführt und bei 70 °C und 85 bar Wasserstoffdruck 16 h hydriert. Das Gemisch wurde abgekühlt, entspannt und im Vakuum vom Lösungsmittel befreit. Der Rückstand wurde mit 650 ml Isopropylacetat aufgenommen, mit 200 ml 1N HCl und mit 100 ml gesättigter NaCl-Lösung gewaschen, auf ein Volumen von 500 ml eingeengt und mit einer Lösung von 19,7 g Natriumacetat in 163 ml Methanol versetzt. Nach ca. 1 h begann die Kristallisation des Produktes. Nach zweistündigem Rühren wurde das Produkt abgesaugt, mit 40 ml Isopropylacetat gewaschen und im Vakuum bei 40°C getrocknet. Es wurden 29.6 g (64% d. Th., 92,7% ee) des (S)-3-(4-Hydroxyphenyl)-2-methoxypropionsäure-Natrium-Salzes als weißer kristalliner Feststoff erhalten.

Durch Umkristallisation aus Methanol/Ethanol konnte die Enantiomerenreinheit des Na-Salzes zusätzlich gesteigert werden. Dazu wurden 28 g des Na-Salzes in 700 ml Ethanol und 350 ml Methanol suspendiert und aus der Suspension 700 ml Lösungsmittel abdestilliert. Anschließend wurde das Gemisch auf Raumtemperatur abgekühlt, filtriert und im Vakuum getrocknet: Es wurden 24 g des Na-Salzes als weißer kristalliner Feststoff (87% d. Th., 97.4% ee) erhalten.
¹H-NMR (400 MHz, D₂O) δ = 2.83 (dd, 1H, CH₂); 2.96 (dd, 1H, CH₂); 3.27 (s, 3H, O-CH₃); 3.87 (dd, 1H, O-CH); 6.85 (dt, 2H, Ar-H); 7.18 (dt, 2H, Ar-H).

### Vergleichsbeispiel 1:

### Herstellung von (S)-3-(4-Hydroxyphenyl)-2-methoxpropionsäure-Natrium-Salz durch Hydrierung mit Rhodium-Katalysator:

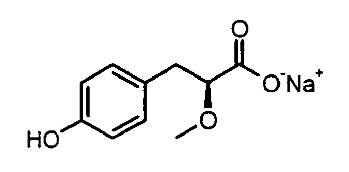

6.3 mg (0.0154 mmol) Rh(COD)₂BF₄ und 6.8 mg (0.0154 mmol) (*S,S*)-2,4-Bis-diphenylphosphanylpentan wurden in 1 ml entgastem Methanol gelöst und zu einer Lösung von 100 mg (0.51 mmol) Z-4'-Hydroxy-2-methoxyzimtsäure in 3 ml entgastem Methanol gegeben. Anschließend wurde das Gemisch in einen Autoklaven überführt und 16 h bei 50°C und 3 bar Wasserstoffdruck hydriert. Das Gemisch wurde auf Raumtemperatur abgekühlt, entspannt und im Vakuum vom Lösungsmittel befreit. Die (*S*)-3-(4-Hydroxyphenyl)-2-methoxypropionsäure wurde mit einem Enantiomerenüberschuß von 80% (100% Umsatz des Substrates) als Öl erhalten.

### Vergleichsbeispiel 2:

### Herstellung von (S)-3-(4-Hydroxvphenyl)-2-methoxvpropionsäure-Natrium-Salz durch Hydrierung mit Iridium-Katalysator:

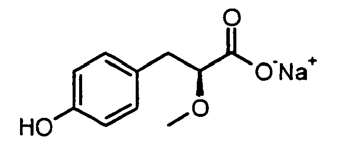

339 mg (0,5 mmol) [Ir(COD)Cl]₂ und 445 mg (1,0 mmol) (*S,S*)-2,4-Bis-diphenylphosphanylpentan wurden in 80 ml entgastem Methanol gelöst und zu einer entgasten Lösung von 40,16 g ( 200,0 mmol) Z-4'-Hydroxy-2-methoxyzimtsäure in 240 ml Isopropylacetat und 60 ml Methanol gegeben. Anschließend wurde das Gemisch in einen Autoklaven überführt und 24 h bei 65°C und 3 bar Wasserstoffdruck hydriert. Das Gemisch wurde auf Raumtemperatur abgekühlt, entspannt und im Vakuum vom Lösungsmittel befreit. Der Rückstand wurde in 400 ml Isopropylacetat aufgenommen und mit einer Lösung von 16,2 g (200 mmol) Natriumacetat in 143 ml Methanol versetzt. Nach ca. 1 h begann die Kristallisation des Produktes. Nach zweistündigem Rühren wurde das Produkt abgesaugt, mit Isopropylacetat nachgewaschen und anschließend im Vakuum bei 40°C getrocknet. Es wurden 25,6 g (53 % d. Th., 92 % ee) des (*S*)-3-(4-Hydroxyphenyl)-2-methoxypropionsäure-Natrium-Salzes als weißer kristalliner Feststoff erhalten.

Durch Umkristallisation aus Methanol/Ethanol konnte die Enantiomerenreinheit des Na-Salzes gesteigert werden. Dazu wurden 25 g des Na-Salzes in 600 ml Ethanol und 300 ml Methanol suspendiert und aus der Suspension 600 ml Lösungsmittel abdestilliert. Anschließend wurde das Gemisch auf Raumtemperatur abgekühlt, filtriert und im Vakuum getrocknet. Es wurden 19 g des Na-Salzes als weißer kristalliner Feststoff (80% d. Th., 97.9% ee) erhalten.

**Tab. 1 Zusammenfassung der Ergebnisse aus Beispiel 2 und Vergleichsbeispiel 1 und 2**

| **Übergangsmetallverbindung** | [RuCl₂(p-Cymol)]₂ | Rh(COD)₂BF₄ | [Ir(COD)Cl]₂ |
|---|---|---|---|
| **Ligand** | (+)-ClMeOBiPHEP | (S,S)-BDPP | (S,S)-BDPP |
| **Substrat/Katalysator-Verhältnis** | 1000 | 100 | 200 |
| **Lösungsmittel** | Methanol | Methanol | Isopropylacetat/ Methanol |
| **Additiv** | Triethylamin 1 eq | - | - |
| **Temperatur** | 70 °C | 50 °C | 65 °C |
| **H₂-Druck** | 85 bar | 3 bar | 3 bar |
| **Reaktionszeit** | 16 h | 24 h | 24 h |
| **ee (in Lösung)** | 78 % | 80 % | 90 % |
| **ee (nach 1. Kristallisation)** | 92 % | Nicht bestimmt | 92% |
| **ee (nach 2. Kristallisation)** | 97 % | | 97% |
| **Ausbeute** | 56 % | Nicht bestimmt | 43 % |

## Patentansprüche

1. Verfahren zur Herstellung von enantiomerenangereicherten Verbindungen der allgemeinen Formel (I), 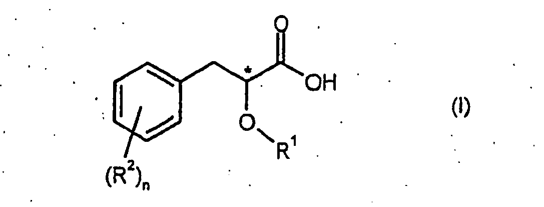 worin
R¹ für einen gegebenenfalls substituierten C₁-C₁₈-Alkylrest, einen gegebenenfalls substituierten C₄-C₂₄-Arylrest oder einen gegebenenfalls substituierten C₅-C₁₈-Arylalkylrest steht,
R² unabhängig voneinander für OH, Halogen, Pseudohalogen, Amino, einen gegebenenfalls substituierten C₁-C₁₈-Alkylrest, einen gegebenenfalls substituierten C₁-C₁₈-Alkoxyrest, einen gegebenenfalls substituierten C₄-C₂₄-Arylrest, einen gegebenenfalls substituierten C₅-C₁₈-Arylalkylrest, einen gegebenenfalls substituierten C₁-C₁₈-Alkyl-Sulfonylrest, einen gegebenenfalls substituierten C₁-C₁₈-Alkyl-Carboxylrest, einen gegebenenfalls substituierten C₁-C₁₈-Alkyl-Carbonylrest, einen gegebenenfalls substituierten C₁-C₁₈-Mono- oder Dialkylaminorest, einen gegebenenfalls substituierten C₁-C₁₈-Alkylsulfonyl-aminorest oder einen gegebenenfalls substituierten C₁-C₁₈-Acylaminorest steht, und
n für 0 oder eine ganze Zahl von 1 bis 5 steht,
**dadurch gekennzeichnet, dass** Verbindungen der allgemeinen Formel (II), 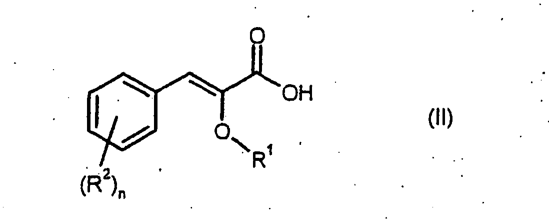 worin
R¹, R² und n die für die allgemeine Formel (I) angegebene Bedeutung haben,
in Gegenwart eines Übergangsmetall-Hydrierkatalysators in Form eines Katalysatorsystems mit Ruthenium-, Rhodium- oder Iridiumverbindungen der Oxidationsstufen Ru(II), Rh(I), Ir(I), jeweils in Kombination mit einem chiralen Diphosphin-Liganden ausgewählt aus (-)- 2,2'-Dichlor-3,3'-dimethoxy-6,6'-bis-diphenylphosphanyl-biphenyl, (-)-3,3'-Bisdiphenylphosphanyl-[4,4']bi(dibenzofuranyl), und dem jeweiligen Enantiomeren enantioselektiv hydriert werden.

2. Verfahren gemäß Anspruch 1, **dadurch gekennzeichnet, dass** die Verbindungen der allgemeinen Formel (II) aus Verbindungen der allgemeinen Formel (III), 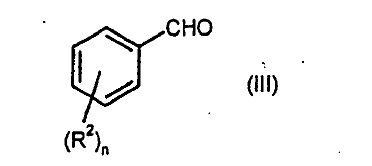 worin
R² und n die in Anspruch 1 genannte Bedeutung haben,
und Verbindungen der allgemeinen Formel (IV), 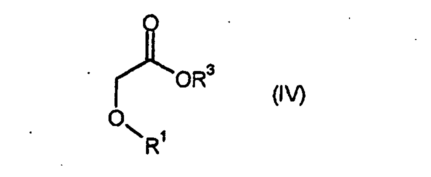 worin
R³ für H oder einen gegebenenfalls substituierten C₁-C₁₈-Alkylrest, bevorzugt einen C₁-C₆-Alkylrest steht und
R¹ die in Anspruch 1 genannte Bedeutung hat,
in einer Abholreaktion in Gegenwart einer Base und gegebenenfalls anschließender Hydrolyse hergestellt werden.

3. Verfahren gemäß Anspruch 1 oder 2, **dadurch gekennzeichnet, dass** R¹ für einen gegebenenfalls substituierten C₁-C₆-Alkylrest, bevorzugt für Methyl steht.

4. Verfahren gemäß wenigstens einem der Ansprüche 1 bis 3, **dadurch gekennzeichnet, dass** R² unabhängig voneinander für OH, F, Cl, Br, CN, einen gegebenenfalls substituierten C₁-C₆-Alkylrest, einen gegebenenfalls substituierten C₆-C₂₄-Arylrest oder einen gegebenenfalls substituierten C₅-C₁₈-Arylalkylrest, bevorzugt für OH steht.

5. Verfahren gemäß wenigstens einem der Ansprüche 1 bis 4, **dadurch gekennzeichnet, dass** n für 1, 2 oder 3, bevorzugt für 1 steht.

6. Verfahren gemäß wenigstens einem der Ansprüche 1 bis 5, **dadurch gekennzeichnet, dass** als Ruthenium-, Rhodium- oder Iridiumverbindung [RuCl₂(p-Cymol)]₂, Ru(COD)Cl₂, [Ru(C₆H₆)Cl₂]₂, Rh(COD)₂BF₄, [Rh(COD)Cl]₂, Rh(NBD)₂BF₄, [Rh(NBD)Cl]₂, Ir(COD)₂BF₄ oder [Ir(COD)Cl]₂ eingesetzt werden.

7. Verfahren gemäß wenigstens einem der Ansprüche 1 bis 6, **dadurch gekennzeichnet, dass** die enantioselektive Hydrierung in Gegenwart wenigstens eines protischen Lösungsmittels durchgeführt wird.

8. Verfahren gemäß Anspruch 7, **dadurch gekennzeichnet, dass** als Lösungsmittel Alkohole, Carbonsäureester, halogenierte aliphatische Kohlenwasserstoffe, cyclische oder aliphatische Ether, cyclische oder aliphatische Kohlenwasserstoffe, aromatische Kohlenwasserstoffe, oder Mischungen aus diesen oder enthaltend einen oder mehrere von diesen eingesetzt werden.

9. Verfahren gemäß wenigstens einem der Ansprüche 1 bis 8, **dadurch gekennzeichnet, dass** die enantioselektive Hydrierung bei Temperaturen von 10°C bis 120°C durchgeführt wird.

10. Verfahren gemäß wenigstens einem der Ansprüche 1 bis 9, **dadurch gekennzeichnet, dass** die enantioselektive Hydrierung bei 1 bis 200 bar Wasserstoffdruck durchgeführt wird.

11. Verfahren gemäß wenigstens einem der Ansprüche 2 bis 10, **dadurch gekennzeichnet, dass** als Base in der Aldolreaktion Alkali- oder Erdalkalimetall-C₁-C₁₈-Alkoxide oder gegebenenfalls substituierte Alkali- oder Erdalkalimetall-C₁-C₁₈-Amide eingesetzt werden.

12. Verfahren gemäß wenigstens einem der Ansprüche 2 bis 11, **dadurch gekennzeichnet, dass** die Aldolreaktion in Gegenwart wenigstens eines Lösungsmittels durchgeführt wird.

13. Verfahren gemäß Anspruch 12, **dadurch gekennzeichnet, dass** als Lösungsmittel Alkohole, cyclische oder offenkettige Ether, aromatische Kolenwasserstoffe, cyclische oder aliphatische Kohlenwasserstoffe oder Mischungen aus diesen oder enthaltend einen oder mehrere von diesen eingesetzt werden.

14. Verfahren gemäß wenigstens einem der Ansprüche 2 bis 13, **dadurch gekennzeichnet, dass** die Aldolreaktion bei Temperaturen von 10°C bis 120°C durchgeführt wird.

15. Verfahren gemäß wenigstens einem der Ansprüche 1 bis 14, **dadurch gekennzeichnet, dass** die Verbindungen der allgemeinen Formel (I) ohne Zwischenisolierung in Gegenwart eines Alkalimetall- oder Ammoniumsalzes direkt als Salze der allgemeinen Formel (V), 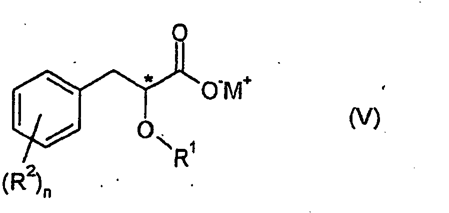 worin
M⁺ für ein Alkalimetallkation oder Ammonium, bevorzugt für Na⁺ steht und
R¹, R² und n die in wenigstens einem der Ansprüche 1 bis 5 angegebene Bedeutung haben, auskristallisiert werden.

## Claims

1. Process for preparing enantiomerically enriched compounds of the general formula (I) 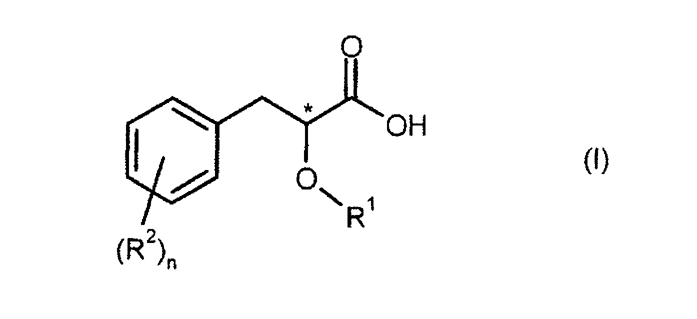 where
R¹ is an optionally substituted C₁-C₁₈-alkyl radical, an optionally substituted C₄-C₂₄-aryl radical or an optionally substituted C₅-C₁₈-arylalkyl radical,
R² are each independently OH, halogen, pseudohalogen, amino, an optionally substituted C₁-C₁₈-alkyl radical, an optionally substituted C₁-C₁₈-alkoxy radical, an optionally substituted C₄-C₂₄-aryl radical, an optionally substituted C₅-C₁₈-arylalkyl radical, an optionally substituted C₁-C₁₈-alkylsulphonyl radical, an optionally substituted C₁-C₁₈-alkylcarboxyl radical, an optionally substituted C₁-C₁₈-alkylcarbonyl radical, an optionally substituted C₁-C₁₈-mono- or dialkylamino radical, an optionally substituted C₁-C₁₈-alkylsulphonylamino radical or an optionally substituted C₁-C₁₈-acylamino radical, and
n is 0 or an integer from 1 to 5,
**characterized in that** compounds of the general formula (II) 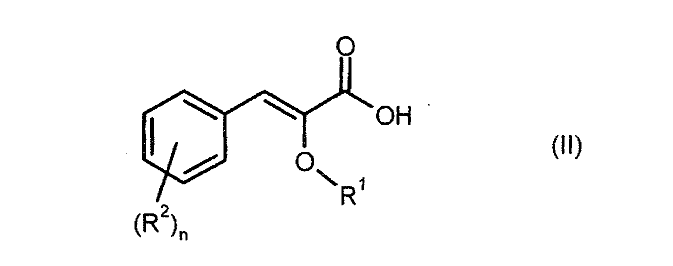 where
R¹, R² and n are each as defined for the general formula (I)
are hydrogenated enantioselectively in the presence of a transition metal hydrogenation catalyst in the form of a catalyst system comprising ruthenium, rhodium or iridium compounds of the Ru(II), Rh(I), Ir(I) oxidation states, in each case in combination with a chiral diphosphine ligand selected from (-)-2,2'-dichloro-3,3'-dimethoxy 6,6'-bis(diphenylphosphinyl)biphenyl, (-)-3,3'-bis(diphenylphosphinyl)-[4,4']bi(dibenzofuranyl), and the particular enantiomer.

2. Process according to Claim 1, **characterized in that** the compounds of the general formula (II) are prepared from compounds of the general formula (III) 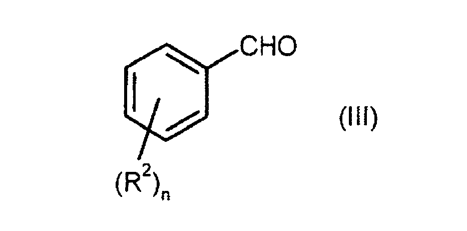 where
R² and n are each as defined in Claim 1,
and compounds of the general formula (IV) 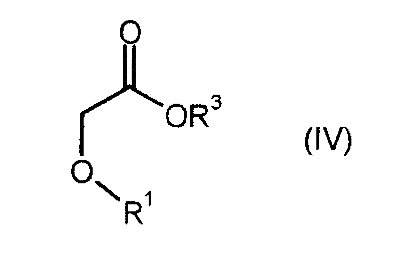 where
R³ is H or an optionally substituted C₁-C₁₈-alkyl radical, preferably a C₁-C₆-alkyl radical, and
R¹ is as defined in Claim 1
in an aldol reaction in the presence of a base, and subsequent hydrolysis if appropriate.

3. Process according to Claim 1 or 2, **characterized in that** R¹ is an optionally substituted C₁-C₆-alkyl radical, preferably methyl.

4. Process according to at least one of Claims 1 to 3, **characterized in that** R² is independently OH, F, Cl, Br, CN, an optionally substituted C₁-C₆-alkyl radical, an optionally substituted C₆-C₂₄-aryl radical or an optionally substituted C₅-C₁₈-arylalkyl radical, preferably OH.

5. Process according to at least one of Claims 1 to 4, **characterized in that** n is 1, 2 or 3, preferably 1.

6. Process according to at least one of Claims 1 to 5, **characterized in that** the ruthenium, rhodium or iridium compound used is [RuCl₂(p-cymene)]₂, Ru(COD)Cl₂, [Ru(C₆Hg)Cl₂]₂, Rh(COD)₂BF₄, [Rh(COD)Cl]₂, Rh(NBD)₂BF₄, [Rh(NBD)Cl]₂, Ir(COD)₂BF₄ or [Ir(COD)Cl]₂.

7. Process according to at least one of Claims 1 to 6, **characterized in that** the enantioselective hydrogenation is performed in the presence of at least one protic solvent.

8. Process according to Claim 7, **characterized in that** the solvents used are alcohols, carboxylic esters, halogenated aliphatic hydrocarbons, cyclic or aliphatic ethers, cyclic or aliphatic hydrocarbons, aromatic hydrocarbons, or mixtures thereof or comprising one or more thereof.

9. Process according to at least one of Claims 1 to 8, **characterized in that** the enantioselective hydrogenation is performed at temperatures of 10°C to 120°C.

10. Process according to at least one of Claims 1 to 9, **characterized in that** the enantioselective hydrogenation is performed at 1 to 200 bar of hydrogen pressure.

11. Process according to at least one of Claims 2 to 10, **characterized in that** the base used in the aldol reaction comprises alkali metal or alkaline earth metal C₁-C₁₈-alkoxides or optionally substituted alkali metal or alkaline earth metal C₁-C₁₈-amides.

12. Process according to at least one of Claims 2 to 11, **characterized in that** the aldol reaction is performed in the presence of at least one solvent.

13. Process according to Claim 12, **characterized in that** the solvents used are alcohols, cyclic or open-chain ethers, aromatic hydrocarbons, cyclic or aliphatic hydrocarbons, or mixtures thereof or comprising one or more thereof.

14. Process according to at least one of Claims 2 to 13, **characterized in that** the aldol reaction is performed at temperatures of 10°C to 120°C.

15. Process according to at least one of Claims 1 to 14, **characterized in that** the compounds of the general formula (I) are crystallized out without intermediate isolation in the presence of an alkali metal or ammonium salt directly as salts of the general formula (V) 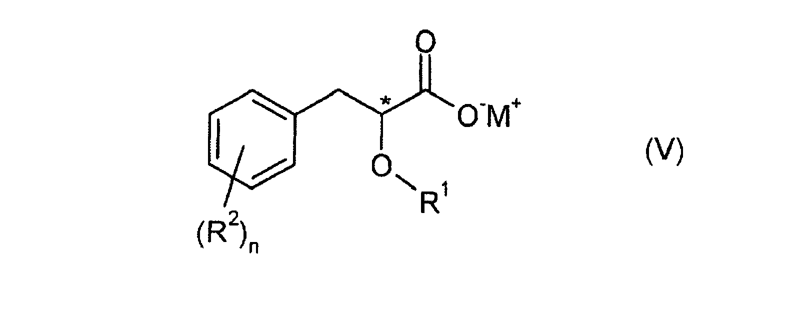 where
M⁺ is an alkali metal cation or ammonium, preferably Na⁺, and
R¹, R² and n are each as defined in at least one of Claims 1 to 5

## Revendications

1. Procédé pour la préparation de composés enrichis en énantiomères de formule générale (I) 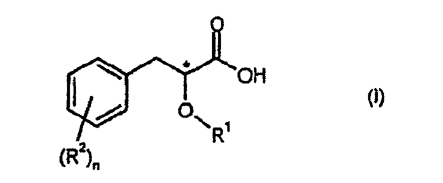 où
R¹ représente un radical C₁-C₁₈-alkyle le cas échéant substitué, un radical C₄-C₂₄-aryle le cas échéant substitué ou un radical C₅-C₁₈-arylalkyle le cas échéant substitué,
R² représente, indépendamment l'un de l'autre, OH, halogène, pseudohalogène, amino, un radical C₁-C₁₈-alkyle le cas échéant substitué, un radical C₁-C₁₈-alcoxy le cas échéant substitué, un radical C₄-C₂₄-aryle le cas échéant substitué, un radical C₅-C₁₈-arylalkyle le cas échéant substitué, un radical C₁-C₁₈-alkylsulfonyle le cas échéant substitué, un radical C₁-C₁₈-alkylcarboxyle le cas échéant substitué, un radical C₁-C₁₈-alkylcarbonyle le cas échéant substitué, un radical C₁-C₁₈-monoalkylamino ou C₁-C₁₈-dialkylamino le cas échéant substitué, un radical C₁-C₁₈-alkylsulfonylamino le cas échéant substitué ou un radical C₁-C₁₈-acylamino le cas échéant substitué, et
n vaut 0 ou un nombre entier de 1 à 5,
**caractérisé en ce qu'**on hydrogène de manière énantiosélective des composés de formule générale (II) 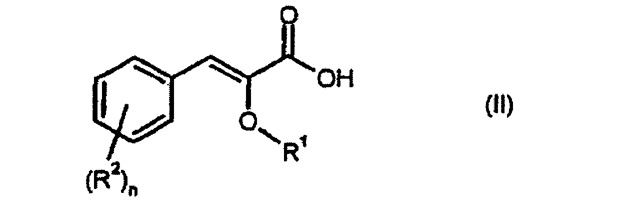 où
R¹, R² et n présentent la signification indiquée pour la formule générale (I)
en présence d'un catalyseur d'hydrogénation à base d'un métal de transition sous forme d'un système de catalyseur avec des composés de ruthénium, de rhodium ou d'iridium présentant les étages d'oxydation Ru(II), Rh(I), Ir(I), à chaque fois en combinaison avec un ligand diphosphine chiral choisi parmi le (-)-2,2'-dichloro-3,3'-diméthoxy-6,6'-bis-diphénylphosphanylbiphényle, le (-)-3,3'-bis-diphénylphosphanyl-[4,4']bi(dibenzofurannyle), et l'énantiomère en question.

2. Procédé selon la revendication 1, **caractérisé en ce que** les composés de formule générale (II) sont préparés à partir de composés de formule générale (III), 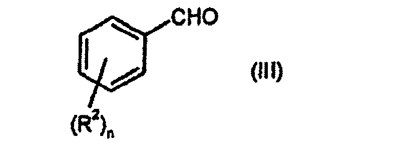 où
R² et n ont la signification mentionnée dans la revendication 1,
et de composés de formule générale (IV) 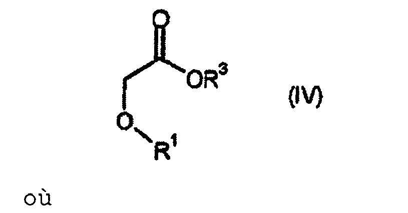
R³ représente H ou un radical C₁-C₁₈-alkyle le cas échéant substitué, de préférence un radical C₁-C₆-alkyle et
R¹ a la signification mentionnée dans la revendication 1,
dans une réaction d'aldol en présence d'une base et le cas échéant une hydrolyse consécutive.

3. Procédé selon la revendication 1 ou 2, **caractérisé en ce que** R¹ représente un radical C₁-C₆-alkyle le cas échéant substitué, de préférence méthyle.

4. Procédé selon au moins l'une quelconque des revendications 1 à 3, **caractérisé en ce que** R² représente, indépendamment l'un de l'autre, OH, F, Cl, Br, CN, un radical C₁-C₆-alkyle le cas échéant substitué, un radical C₆-C₂₄-aryle le cas échéant substitué ou un radical C₅-C₁₈-arylalkyle le cas échéant substitué, de préférence OH.

5. Procédé selon au moins l'une quelconque des revendications 1 à 4, **caractérisé en ce que** n vaut 1, 2 ou 3, de préférence 1.

6. Procédé selon au moins l'une quelconque des revendications 1 à 5, **caractérisé en ce qu'**on utilise comme composé à base de ruthénium, de rhodium ou d'iridium, [RuCl₂(p-Cymol)]₂, Ru(COD)Cl₂, [Ru((C₆H₆)Cl₂]₂, Rh(COD)₂BF₄, [Rh(COD)Cl]₂, Rh(NBD)₂BF₄, [Rh(NBD)Cl]₂, Ir(COD)₂BF₄ ou [Ir(COD)Cl]₂.

7. Procédé selon au moins l'une quelconque des revendications 1 à 6, **caractérisé en ce que** l'hydrogénation énantiosélective est réalisée en présence d'au moins un solvant protique.

8. Procédé selon la revendication 7, **caractérisé en ce qu'**on utilise comme solvants des alcools, des esters d'acides carboxyliques, des hydrocarbures aliphatiques halogénés, des éthers cycliques ou aliphatiques, des hydrocarbures cycliques ou aliphatiques, des hydrocarbures aromatiques, ou des mélanges de ceux-ci ou contenant un ou plusieurs de ceux-ci.

9. Procédé selon au moins l'une quelconque des revendications 1 à 8, **caractérisé en ce que** l'hydrogénation énantiosélective est réalisée à des températures de 10 à 120°C.

10. Procédé selon au moins l'une quelconque des revendications 1 à 9, **caractérisé en ce que** l'hydrogénation énantiosélective est réalisée à une pression d'hydrogène de 1 à 200 bars.

11. Procédé selon au moins l'une quelconque des revendications 2 à 10, **caractérisé en ce qu'**on utilise comme base dans la réaction d'aldol des C₁-C₁₈-alcoxydes de métal alcalin ou alcalino-terreux ou des C₁-C₁₈-amides de métal alcalin ou alcalino-terreux le cas échéant substitués.

12. Procédé selon au moins l'une quelconque des revendications 2 à 11, **caractérisé en ce que** la réaction d'aldol est réalisée en présence d'au moins un solvant.

13. Procédé selon la revendication 12, **caractérisé en ce qu'**on utilise comme solvants des alcools, des éthers cycliques ou à chaîne ouverte, des hydrocarbures aromatiques, des hydrocarbures cycliques ou aliphatiques ou des mélanges de ceux-ci ou contenant un ou plusieurs de ceux-ci.

14. Procédé selon au moins l'une quelconque des revendications 2 à 13, **caractérisé en ce que** la réaction d'aldol est réalisée à des températures de 10 à 120°C.

15. Procédé selon au moins l'une quelconque des revendications 1 à 14, **caractérisé en ce que** les composés de formule générale (I) sont séparés par cristallisation sans isolement intermédiaire en présence d'un sel de métal alcalin ou d'ammonium, directement sous forme de sels de formule générale (V), 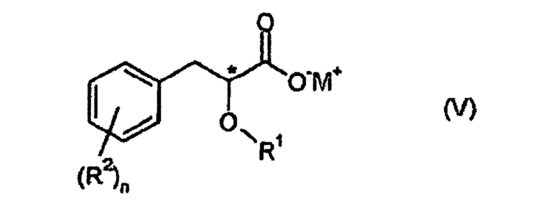 où
M⁺ représente un cation de métal alcalin ou d'ammonium, de préférence Na⁺ et
R¹, R² et n présentent la signification indiquée dans au moins l'une quelconque des revendications 1 à 5.
